# EUROPEAN PATENT APPLICATION

(11) **EP 4 692 860 A1**
(43) Date of publication of application: **11.02.2026**
(21) Application number: 24796474.5
(22) Date of filing: 18.01.2024
(51) Int. Cl.: G01T 7/00, A61B 6/42, H04N 25/70

(54) **RADIOGRAPHIC IMAGING DEVICE**

(30) Priority: 27.04.2023 JP 2023073485; 29.08.2023 JP 2023138714
(71) Applicant: Hamamatsu Photonics K.K., Hamamatsu-shi, Shizuoka 435-8558 (JP)
(72) Inventor: TOYODA Kenta, Hamamatsu-shi, Shizuoka 435-8558 (JP); IWATA Takuya, Hamamatsu-shi, Shizuoka 435-8558 (JP); NAMBA Shuhei, Hamamatsu-shi, Shizuoka 435-8558 (JP); FUKAMIZU Seiji, Hamamatsu-shi, Shizuoka 435-8558 (JP); KYUSHIMA Ryuji, Hamamatsu-shi, Shizuoka 435-8558 (JP); FUJITA Kazuki, Hamamatsu-shi, Shizuoka 435-8558 (JP); SAWADA Junichi, Hamamatsu-shi, Shizuoka 435-8558 (JP); ARITAKE Takao, Hamamatsu-shi, Shizuoka 435-8558 (JP); OKADA Haruyoshi, Hamamatsu-shi, Shizuoka 435-8558 (JP); TAKASE Yusuke, Hamamatsu-shi, Shizuoka 435-8558 (JP)
(74) Representative: Hoffmann Eitle
(86) International application number: PCT/JP2024/001338
(87) International publication number: WO 2024/224715

(57) **Abstract**

A radiation imaging device according to an embodiment includes a sensor panel, a control board, a flexible printed circuit that connects the sensor panel and a back surface of the control board through a side of the control board, a circuit board on which a power supply circuit and an interface circuit are mounted, a first housing that houses the sensor panel, the control board, and the flexible printed circuit, a second housing that houses the circuit board, and a connection member that electrically connects the control board and the circuit board. A communication hole provided in the wall portion of the first housing is provided so as not to overlap with a portion of the flexible printed circuit from the end farthest from the control board in the second direction to the end connected to the control board when viewed from the first direction.

## Description

### Technical Field

The present disclosure relates to a radiation imaging device.

### Background Art

There is known a radiation imaging device (X-ray image capturing device) including a sensor panel (X-ray detection sensor), a flexible printed circuit (flexible wiring substrate) connected to the sensor panel, a first housing (first housing) accommodating the sensor panel, a control board (signal processing circuit board) and a circuit board (power supply circuit board) arranged side by side with the sensor panel in a planar direction, and a second housing (second housing) accommodating the control board and the circuit board (see, for example, Patent Document 1). In the radiation imaging device as described above, the sensor panel in the first housing and the control board and the circuit board in the second housing may be connected to each other via a connection member (cable) passing through a communication hole provided in a wall portion of the first housing for transmission and reception of electric signals and the like.

### Citation List

### Patent Literature

Patent Literature 1: Japanese Unexamined Patent Publication No. 2010-281753

### Summary of Invention

### Technical Problem

In the radiation imaging device as described above, an interface circuit that outputs a signal from the control board to the outside may be mounted on the circuit board in addition to the power supply circuit. In this case, noise generated from the power supply circuit and the interface circuit is propagated to the flexible printed circuit through the communication hole provided in the wall of the first housing, and as a result, the noise may be superimposed on an electric signal flowing through the flexible printed circuit. As a result, image quality of a radiation image obtained by the radiation imaging device may be deteriorated, and reliability of the radiation imaging device may be deteriorated. In addition, in the configuration of the radiation imaging device as described above, since the control board and the circuit board are arranged side by side with the sensor panel in the planar direction, the dimensions of the radiation imaging device in a plan view increase.

An object of one aspect of the present disclosure is to provide a radiation imaging device capable of achieving both improvement in reliability and reduction in dimensions in a plan view.

### Solution to Problem

The present disclosure includes the radiation imaging devices according to [1] to [12].
[1] A radiation imaging device comprising:
   a sensor panel configured to detect radiation;
   a control board having a main surface facing the sensor panel along a first direction orthogonal to the sensor panel and a back surface opposite to the main surface, and configured to control reading of a signal from the sensor panel;
   a flexible printed circuit disposed so as to connect the sensor panel and the back surface of the control board through a side of the control board when viewed from the first direction;
   a circuit board with a power supply circuit that supplies power to the control board and an interface circuit that outputs a signal from the control board to the outside mounted thereon;
   a first housing accommodating the sensor panel, the control board, and the flexible printed circuit, and having a wall portion facing the back surface of the control board;
   a second housing provided at a position facing the back surface of the control board with the wall portion interposed therebetween and accommodating the circuit board; and
   at least one connection member electrically connecting the control board and the circuit board, in which
   the wall portion is provided with at least one communication hole that allows the inside of the first housing and the inside of the second housing to communicate with each other,
   the at least one connection member is connected to the control board and the circuit board via the at least one communication hole, and
   when viewed from the first direction, the at least one communication hole is provided so as not to overlap a portion of the flexible printed circuit from an end farthest from the control board in a second direction orthogonal to the first direction to an end connected to the control board.
   In the configuration [1], the main surface of the control board faces the sensor panel, and the second housing accommodating the circuit board faces the back surface of the control board with the wall portion of the first housing interposed therebetween. That is, the control board and the circuit board on which the power supply circuit and the interface circuit are mounted are arranged side by side along the first direction with respect to the sensor panel. As a result, the dimensions in plan view can be reduced as compared with a case where the control board and the circuit board are arranged side by side along the direction perpendicular to the first direction with respect to the sensor panel (second direction). Furthermore, in the configuration of [1], the communication hole provided in the wall portion is provided so as not to overlap with a portion of the flexible printed circuit from the end farthest from the control board in the second direction to the end connected to the control board. As a result, it is possible to suppress noise generated from the power supply circuit and the interface circuit from propagating to the flexible printed circuit via the communication hole. As a result, deterioration in image quality of the radiation image or the like caused by the noise is suppressed, so that the reliability of the radiation imaging device can be improved. Therefore, according to the configuration of [1], it is possible to achieve both improvement in reliability and reduction in dimensions in a plan view.
[2] The radiation imaging device according to [1], in which the at least one communication hole is provided so as not to overlap the entire flexible printed circuit when viewed from the first direction.
   According to the configuration of [2], it is possible to more effectively suppress noise generated from the power supply circuit and the interface circuit from propagating to the flexible printed circuit via the communication hole. Therefore, the reliability of the radiation imaging device can be further improved.
[3] The radiation imaging device according to [1] or [2], in which
   the at least one connection member includes a plurality of connection members, and
   the at least one communication hole includes a plurality of communication holes provided to correspond to the plurality of connection members, respectively.
   According to the configuration of [3], it is possible to suppress noise generated from the power supply circuit and the interface circuit from propagating to the flexible printed circuit via the plurality of communication holes while increasing the amount of signal transmission between the control board and the circuit board.
[4] The radiation imaging device according to any one of [1] to [3], in which
   a first FPGA is mounted on the control board, and
   the at least one communication hole is provided so as not to overlap the first FPGA when viewed from the first direction.
   According to the configuration of [4], it is possible to suppress noise generated from the power supply circuit and the interface circuit from propagating to the first FPGA via the communication hole. As a result, reliability of the first FPGA can be improved, and as a result, malfunction of the first FPGA due to the noise can be suppressed.
[5] The radiation imaging device according to [4], in which the at least one connection member includes a plurality of connection members, and
   the plurality of connection members include:
   a first connection member disposed on a first side with respect to the first FPGA when viewed from the first direction; and
   a second connection member disposed on a second side different from the first side with respect to the first FPGA.
   According to the configuration of [5], since the wiring length between the first FPGA and the first connection member and the wiring length between the first FPGA and the second connection member can be made substantially the same, the timing of signal transmission and reception between the first FPGA and the first connection member and the timing of signal transmission and reception between the first FPGA and the second connection member can be made substantially the same.
[6] The radiation imaging device according to [5] in which the plurality of connection members are arranged to be point-asymmetric with the first FPGA as a center when viewed from the first direction.
   According to the configuration of [6], when the control board and the circuit board are connected to each other by the plurality of connection members, the directions of the control board and the circuit board can be easily specified based on the arrangement of the plurality of connection members. Therefore, the efficiency of the work of connecting the control board and the circuit board via the plurality of connection members can be improved.
[7] The radiation imaging device according to any one of [4] to [6], in which
   a second FPGA is mounted on the circuit board, and
   at least a part of the second FPGA is arranged to overlap the first FPGA when viewed from the first direction.
   According to the configuration of [7], the connection member connecting the control board and the circuit board to each other along the first direction can be connected to the back surface of the control board in the vicinity of the first FPGA and can be connected to the circuit board in the vicinity of the second FPGA. Accordingly, since the wiring length between the first FPGA and the second FPGA can be shortened, it is possible to suppress the occurrence of delay or rounding of the transmission signal waveform between the first FPGA and the second FPGA.
[8] The radiation imaging device according to any one of [1] to [7], in which the at least one communication hole is arranged outside a region linearly connecting the flexible printed circuit and the power supply circuit.
   According to the configuration of [8], it is possible to more effectively suppress noise generated from the power supply circuit from propagating to the flexible printed circuit via the communication hole. As a result, the reliability of the radiation imaging device can be further improved.
[9] The radiation imaging device according to any one of [1] to [8], further comprising: a connector provided on the circuit board and connected to the outside, in which
   the second housing is provided with an opening for exposing the connector to the outside of the second housing, and
   the connector is located inside an outer edge of the first housing when viewed from the first direction.
   According to the configuration of [9], the radiation imaging device (circuit board) can be connected to the external device via the connector exposed to the outside via the opening of the second housing. Furthermore, since the connector is located inside the outer edge of the first housing when viewed from the first direction, it is possible to suppress an increase in dimensions of the radiation imaging device in plan view while adopting the configuration in which the connector is exposed from the second housing as described above.
[10] The radiation imaging device according to [9], in which
   the second housing has a side surface portion extending in the first direction and provided with the opening, and
   the side surface portion is located inside an outer edge of the first housing when viewed from the first direction.
   According to the configuration of [10] described above, since the side surface portion provided with the opening is located inside the outer edge of the first housing when viewed from the first direction, it is possible to suppress an increase in dimensions of the radiation imaging device in plan view while adopting the configuration in which the connector is exposed from the side surface portion. As a result, the cable connected to the connector can be easily routed.
[11] The radiation imaging device according to any one of [1] to [10], in which the circuit board is fixed to the first housing such that a ground electrode provided on the circuit board is electrically connected to the first housing.
   According to the configuration of [11], when the first housing is set to the ground potential, the circuit board can be easily connected to GND.
[12] The radiation imaging device according to any one of [1] to [11], in which the circuit board is provided so as not to overlap the flexible printed circuit when viewed from the first direction.

According to the configuration of [12], it is possible to more effectively suppress noise generated from the power supply circuit and the interface circuit from propagating to the flexible printed circuit. As a result, the reliability of the radiation imaging device can be further improved.

### Advantageous Effects of Invention

According to one aspect of the present disclosure, it is possible to provide a radiation imaging device capable of achieving both improvement in reliability and reduction in dimensions in a plan view.

### Brief Description of Drawings

FIG. 1 is a perspective view of a radiation imaging device according to a first embodiment.
FIG. 2 is an exploded perspective view of the radiation imaging device.
FIG. 3 is an exploded perspective view of the radiation imaging device.
FIG. 4 is a cross-sectional view of the radiation imaging device taken along line IV-IV in FIG. 1.
FIG. 5 is a cross-sectional view of the radiation imaging device taken along line V-V in FIG. 1.
FIG. 6 is a cross-sectional view of the radiation imaging device taken along line VI-VI in FIG. 4.
FIG. 7 is a cross-sectional view of a radiation imaging device according to a second embodiment.
FIG. 8 is a cross-sectional view of the radiation imaging device according to a second embodiment.
FIG. 9 is a cross-sectional view of the radiation imaging device taken along line IX-IX in FIG. 7.

### Description of Embodiments

Hereinafter, an embodiment of the present disclosure will be described in detail with reference to the drawings. Note that, in the following description, identical or equivalent elements are denoted by identical reference signs, and redundant description thereof will be omitted. In addition, in the drawings, there are some parts exaggerated for easy understanding of characteristic parts according to the embodiments. Thus, dimensional ratios of each part in the drawings may be different from actual dimensional ratios.

### [First Embodiment]

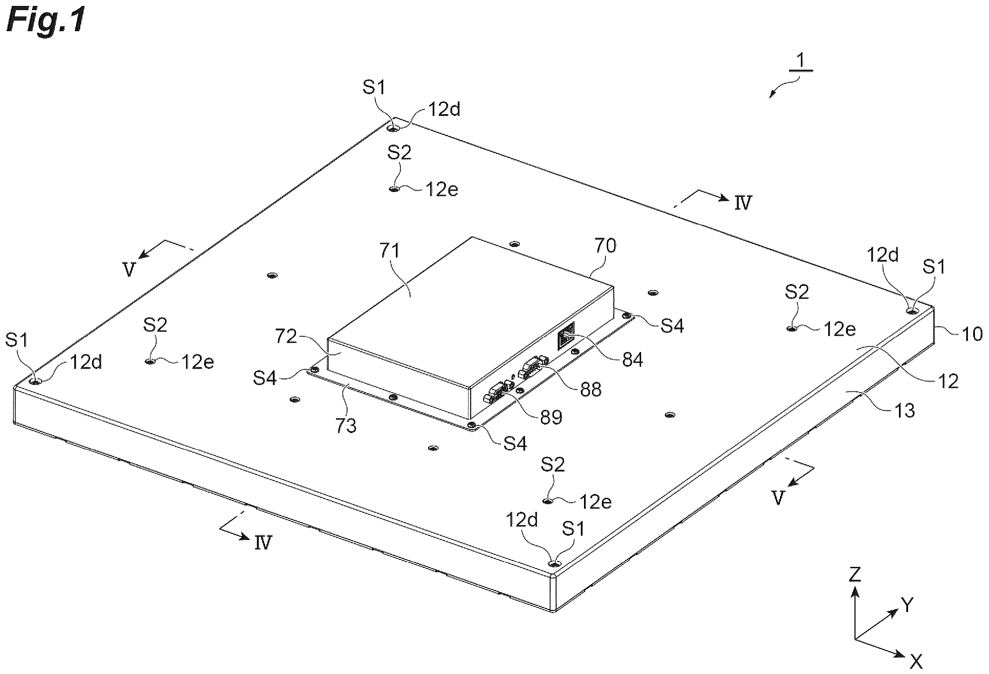

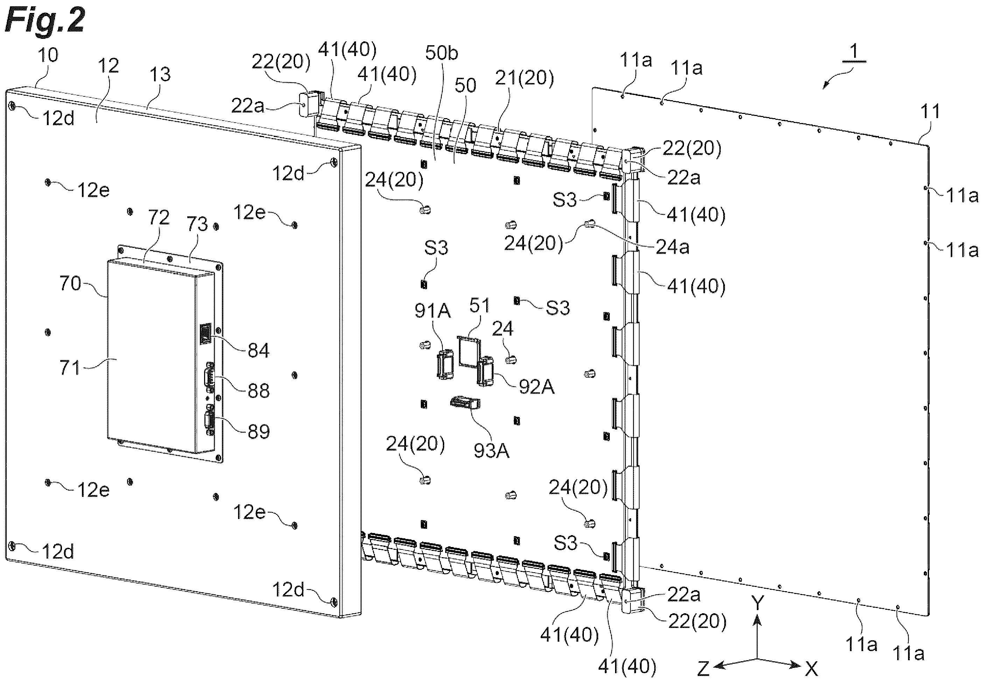

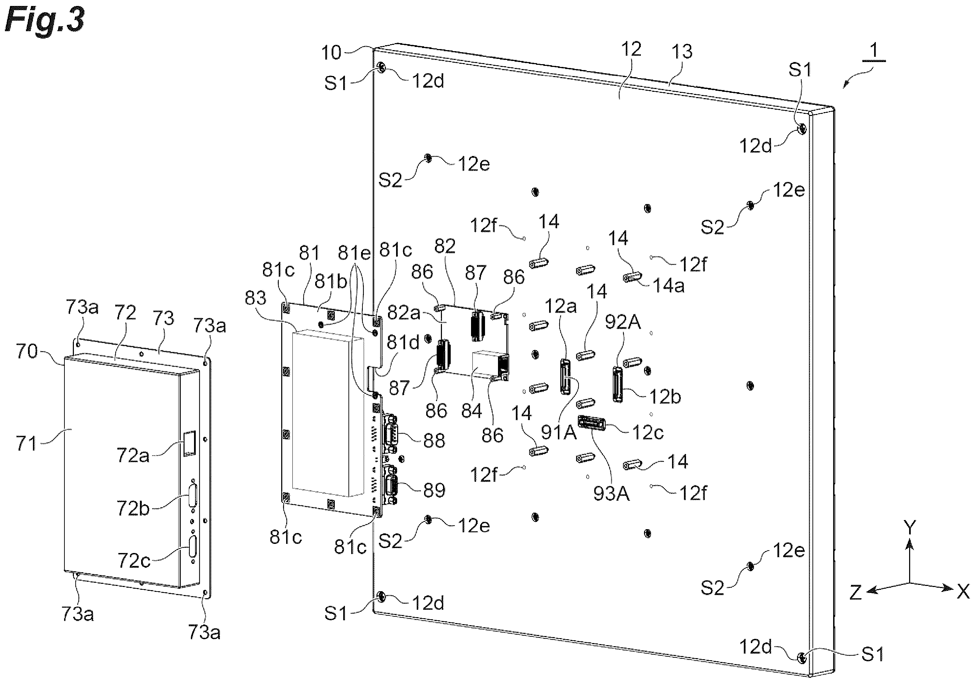

A configuration of a radiation imaging device 1 according to a first embodiment will be described with reference to FIGS. 1 to 5. The radiation imaging device 1 is, for example, a large-area flat panel sensor (X-ray imaging device) used in a medical X-ray imaging system. As illustrated in FIG. 1, the radiation imaging device 1 includes a first housing 10 having a substantially rectangular parallelepiped shape and a second housing 70 having a substantially rectangular parallelepiped shape and fixed to the first housing 10. As illustrated in FIGS. 2 and 3, each member constituting the radiation imaging device 1 is accommodated in the first housing 10 and the second housing 70.

The first housing 10 is a hollow container having a substantially rectangular parallelepiped outer shape. As illustrated in FIGS. 3 to 5, first housing 10 includes a top wall 11, a bottom wall 12 (wall portion), a side wall 13, and a plurality of pillars 14. The top wall 11 and the bottom wall 12 are both formed in a rectangular plate shape, and are arranged to face each other while being separated from each other. In the present specification, a direction in which the top wall 11 and the bottom wall 12 face each other (a direction orthogonal to a sensor panel 30 to be described later) is referred to as a Z-axis direction (first direction). A direction orthogonal to the Z-axis direction (in the present embodiment, the lateral direction of the radiation imaging device 1 when viewed from the Z-axis direction) is referred to as an X-axis direction (second direction), and a direction orthogonal to the Z-axis direction and the X-axis direction (in the present embodiment, the longitudinal direction of the radiation imaging device 1 when viewed from the Z-axis direction) is referred to as a Y-axis direction (second direction). The radiation imaging device 1 is disposed such that radiation to be detected (in the present embodiment, an X-ray is used) enters the top wall 11 along the Z-axis direction. When the radiation passes through the top wall 11 and enters a sensor panel 30 to be described later, the radiation is detected in the radiation imaging device 1.

The top wall 11 is a rectangular plate-shaped member. The top wall 11 is made of, for example, a material that transmits radiation (for example, carbon fiber). The top wall 11 guides the radiation incident along the Z-axis direction to the inside of the first housing 10. The top wall 11 is located on a side on which radiation is incident. As illustrated in FIG. 2, a plurality of (in the present embodiment, 32) through holes 11a are provided in a portion along the outer edge portion of the top wall 11.

The bottom wall 12 is a rectangular plate-shaped member. The bottom wall 12 is formed of, for example, a material that shields radiation. In addition, the bottom wall 12 is formed of a material that shields noise (for example, radiation noise) generated from the power supply circuit 83 and the interface circuit 84 to be described later. For example, the bottom wall 12 is formed of a conductive metal material (for example, iron, aluminum, stainless steel, and the like). In the present embodiment, the bottom wall 12 is formed of aluminum. The bottom wall 12 is located on the side opposite to the side on which the radiation is incident. As illustrated in FIGS. 3 to 5, a plurality of communication holes 12a, 12b, and 12c (at least one communication hole) are provided in a substantially central portion of the bottom wall 12 when viewed from the Z-axis direction. The communication holes 12a to 12c are holes penetrating the bottom wall 12 in the Z-axis direction so as to communicate the inside of the first housing 10 and the inside of the second housing 70. In the present embodiment, the communication holes 12a to 12c are formed in a substantially rectangular shape when viewed from the Z-axis direction. The communication holes 12a and 12b are provided to face each other while being separated from each other in the X-axis direction. The communication hole 12c is located between the communication holes 12a and 12b in the X-axis direction. The communication hole 12c is separated from the communication holes 12a and 12b toward one side (lower side in FIG. 3) in the Y-axis direction. The communication holes 12a, 12b, and 12c are provided to correspond to the connection members 91, 92, and 93 described later, respectively. That is, in the present embodiment, the connection members 91, 92, and 93 independently pass through the communication holes 12a, 12b, and 12c, respectively.

The bottom wall 12 is provided with a plurality of (four in the present embodiment, 4) through holes 12d, a plurality of (in the present embodiment, 12) through holes 12e, and a plurality of (in the present embodiment, 10) screw holes 12f. The through holes 12d are provided at corners (four corners) of the bottom wall 12. The screw holes 12f are provided so as to surround the entire communication holes 12a to 12c in a rectangular shape.

The side wall 13 extends along the XZ plane and the YZ plane, and is formed in a rectangular annular shape so as to connect the outer edge portion of the top wall 11 and the outer edge portion of the bottom wall 12. The side wall 13 is formed of, for example, a metal material (for example, iron, aluminum, stainless steel, and the like) that shields radiation. In the present embodiment, the side wall 13 is formed of aluminum. The side wall 13 is provided with a plurality of screw holes (not illustrated) at positions, corresponding to the through holes 11a of the top wall 11. Shafts of screws (not illustrated) are inserted into the through holes 11a and screwed into the screw hole of the side wall 13, whereby the top wall 11 and the side wall 13 are fixed to each other.

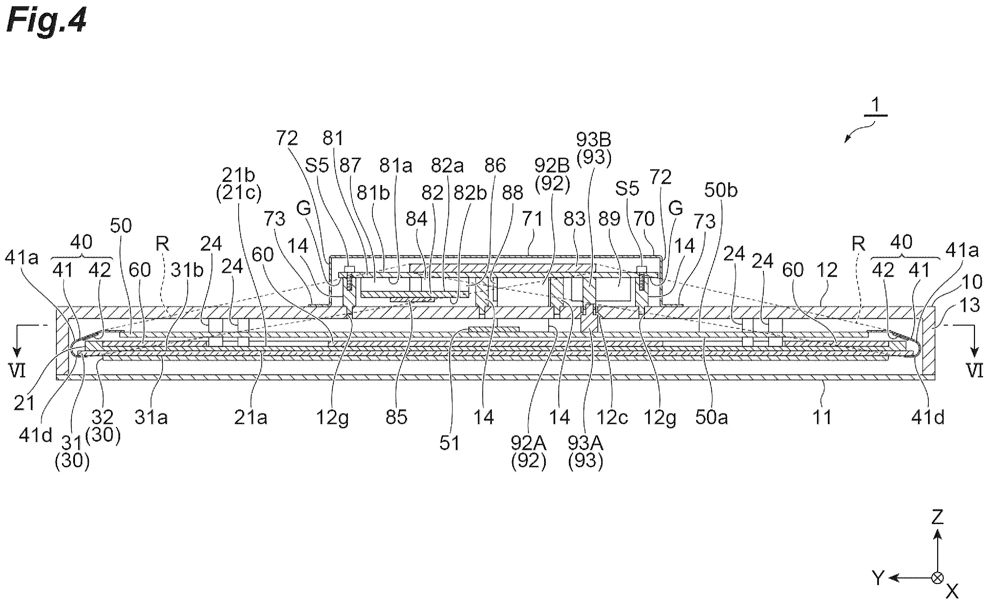

The plurality of (in the present embodiment, 11) pillars 14 are members that fix the circuit board 81 and the bottom wall 12 described later to each other. The pillar 14 is made of, for example, a metal material (for example, iron, aluminum, stainless steel, brass, and the like). In the present embodiment, the pillar 14 is formed of brass. One end portion of the pillar 14 is fixed to the bottom wall 12. Specifically, a screw groove is formed in one end portion of the pillar 14, and the end portion is screwed into a screw hole 12g (see FIG. 4) provided in the bottom wall 12. The pillar 14 is fixed to the bottom wall 12 inside a region surrounded by the screw holes 12f. The pillar 14 is provided with a screw hole 14a into which a shaft of a screw S5 described later is screwed.

As illustrated in FIGS. 2, 4, and 5, the radiation imaging device 1 includes a support member 20, a sensor panel 30, a plurality of (in the present embodiment, 30) flexible circuit boards 40, a control board 50, and a plurality of (in the present embodiment, 3) radiation shielding boards 60 housed in the first housing 10.

The support member 20 is a member for supporting (fixing) the sensor panel 30, the control board 50, the radiation shielding board 60, and the like with respect to the first housing 10. In the present embodiment, the support member 20 includes a base board 21, a plurality of (in the present embodiment, 4) pillars 22, a plurality of (in the present embodiment, 16) pillars 23, and a plurality of (in the present embodiment, 12) pillars 24. The pillars 22 are fixed to corners (four corners) of base board 21 with screws or the like. The pillars 23 and 24 are formed on the back surface 21b of the base board 21.

The base board 21 has a support surface 21a that supports the sensor panel 30 and a back surface 21b opposite to the support surface 21a. The base board 21 is made of, for example, a metal such as iron, aluminum, stainless steel, a tungsten alloy, or copper tungsten. In the present embodiment, as an example, the base board 21 is formed of relatively lightweight aluminum. The support surface 21a is a surface facing the top wall 11, and the back surface 21b is a surface facing the bottom wall 12. The support surface 21a supports the sensor board 31 of the sensor panel 30.

On the back surface 21b of the base board 21, a recessed portion 21c for arranging a plurality of (in the present embodiment, 3) radiation shielding boards 60 is provided. Each radiation shielding board 60 is formed of, for example, a radiation shielding material such as lead (hard lead). In the present embodiment, a rectangular plate-shaped radiation shielding board 60 extending in the X-axis direction is provided at each of the central portion and both side edge portions of the recessed portion 21c in the Y-axis direction. The radiation shielding boards 60 are arranged to be separated from each other in the Y-axis direction. Each radiation shielding board 60 is disposed in the recessed portion 21c, and is adhesively fixed to the bottom surface of the recessed portion 21c via an adhesive member such as a double-sided tape.

The base board 21 is fixed to the first housing 10 via a pillar 22 (see FIG. 2). Specifically, the pillar 22 is provided with a screw hole 22a. Then, a shaft of a screw S1 (see FIG. 3) is inserted into the through hole 12d of the bottom wall 12 and screwed into the screw hole 22a of the pillar 22, whereby the base board 21 and the first housing 10 are fixed to each other. Further, the base board 21 is fixed to the first housing 10 via the pillar 24. Specifically, the pillar 24 penetrates the control board 50, and a screw hole 24a is provided at the tip of the pillar 24. Then, a shaft of a screw S2 (see FIG. 3) is inserted into the through hole 12e of the bottom wall 12 and screwed into the screw hole 24a of the pillar 24, whereby the base board 21 and the first housing 10 are fixed to each other. As a result, the base board 21 is stably supported with respect to the first housing 10.

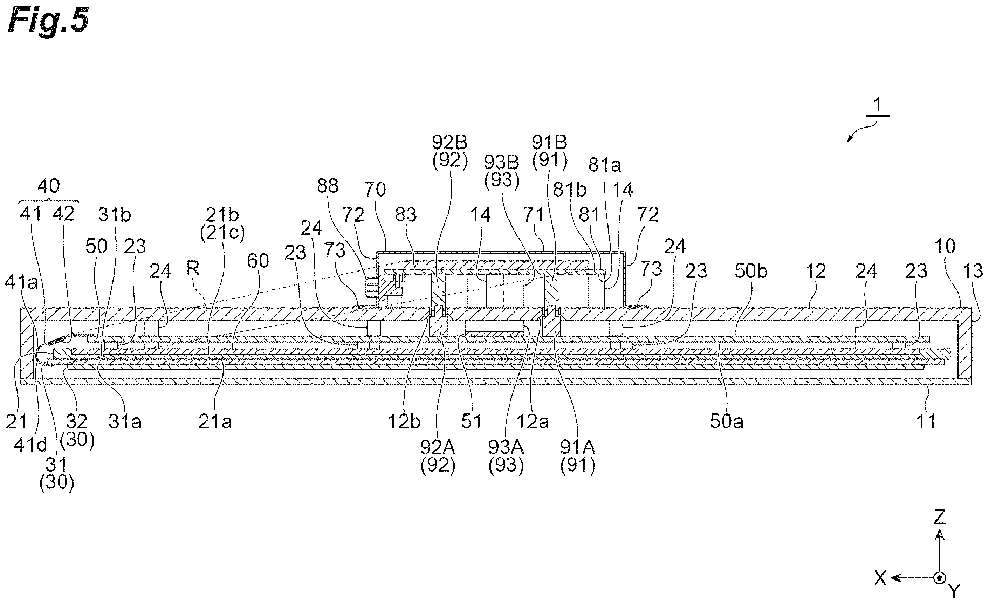

The control board 50 is fixed to the back surface 21b of the base board 21 via the pillar 23 (see FIG. 5). Specifically, a screw hole is provided at the tip of the pillar 23. Then, a shaft of a screw S3 (see FIG. 2) is inserted into a through hole provided in the control board 50 and screwed into a screw hole of the pillar 23, whereby the base board 21 and the control board 50 are fixed to each other.

The sensor panel 30 includes a sensor board 31 formed in a rectangular plate shape and a scintillator 32. The sensor board 31 has a first surface 31a on which a light receiving unit (not illustrated) is formed and a second surface 31b opposite to the first surface 31a. The first surface 31a is a surface facing the top wall 11, and the second surface 31b is a surface facing the bottom wall 12. The scintillator 32 is disposed on the light receiving unit (first surface 31a). The scintillator 32 is formed by, for example, depositing a scintillator material containing CsI as a main component on a light receiving unit. The scintillator 32 converts radiation incident through the top wall 11 into light. Specifically, the scintillator 32 outputs scintillation light having an intensity corresponding to the incident intensity of radiation to the light receiving unit.

The sensor board 31 is, for example, a transparent glass substrate. The sensor board 31 is fixed to the base board 21 by fixing the second surface 31b of the sensor board 31 to the support surface 21a of the base board 21. The second surface 31b is fixed to the support surface 21a via an adhesive member such as a double-sided tape. A plurality of electrode pads (not illustrated) are formed on an outer edge portion on one side (left side in FIG. 5) of the first surface 31a in the X axis direction. A plurality of electrode pads (not illustrated) are also formed on outer edge portions of one side (left side in FIG. 4) and the other side (right side in FIG. 4) of the first surface 31a in the Y axis direction. Each electrode pad is electrically connected to a pixel formed in the light receiving unit via wiring or the like. A corresponding flexible circuit board 40 is connected to each electrode pad.

The flexible circuit board 40 is a circuit member electrically connected to an electrode pad formed on the first surface 31a of the sensor board 31. The flexible circuit board 40 includes a flexible printed circuit 41 that can be deformed, such as bent, and an IC chip 42 mounted on the flexible printed circuit 41.

As illustrated in FIGS. 4 and 5, the flexible printed circuit 41 is disposed so as to connect the first surface 31a of the sensor board 31 (sensor panel 30) and the back surface 50b of the control board 50 through the sides of the sensor panel 30, the support member 20, and the control board 50 when viewed from the Z-axis direction. That is, the flexible printed circuit 41 is curved so as not to interfere with the sensor panel 30, the support member 20, and the control board 50. The flexible printed circuit 41 has, for example, a structure in which a circuit pattern made of a conductor foil (for example, copper or the like) is formed on an insulator (for example, polyimide or the like) on a thin film. The IC chip 42 is a readout circuit (readout IC (ROIC)) that executes processing for reading a signal from the sensor panel 30.

The control board 50 is a circuit board that controls reading of signals from the sensor panel 30. The control board 50 is disposed at a position facing the back surface 21b of the base board 21. The control board 50 has a main surface 50a and a back surface 50b opposite to the main surface 50a. The main surface 50a faces the sensor panel 30 with the support member 20 and the radiation shielding board 60 interposed therebetween along the Z-axis direction. The back surface 50b faces the bottom wall 12 of the first housing 10. As illustrated in FIG. 2, in the present embodiment, connection ports (connectors) for the flexible printed circuit 41 are provided along three sides of the C-shape excluding one side of the outer edge portion (four sides) of the back surface 50b of the control board 50. Specifically, six flexible printed circuits 41 are connected to an outer edge portion of the back surface 50b on one side (right side in FIG. 2) in the X-axis direction at substantially equal intervals. The twelve flexible printed circuits 41 are connected to an outer edge portion of the back surface 50b on one side (upper side in FIG. 2) in the Y-axis direction at substantially equal intervals. The twelve flexible printed circuits 41 are connected to an outer edge portion of back surface 50b on the other side (lower side in FIG. 2) in the Y-axis direction at substantially equal intervals. The light received by the light receiving unit of the sensor board 31 is converted into an electric signal, and the electric signal is transmitted to the control board 50 via the flexible printed circuit 41.

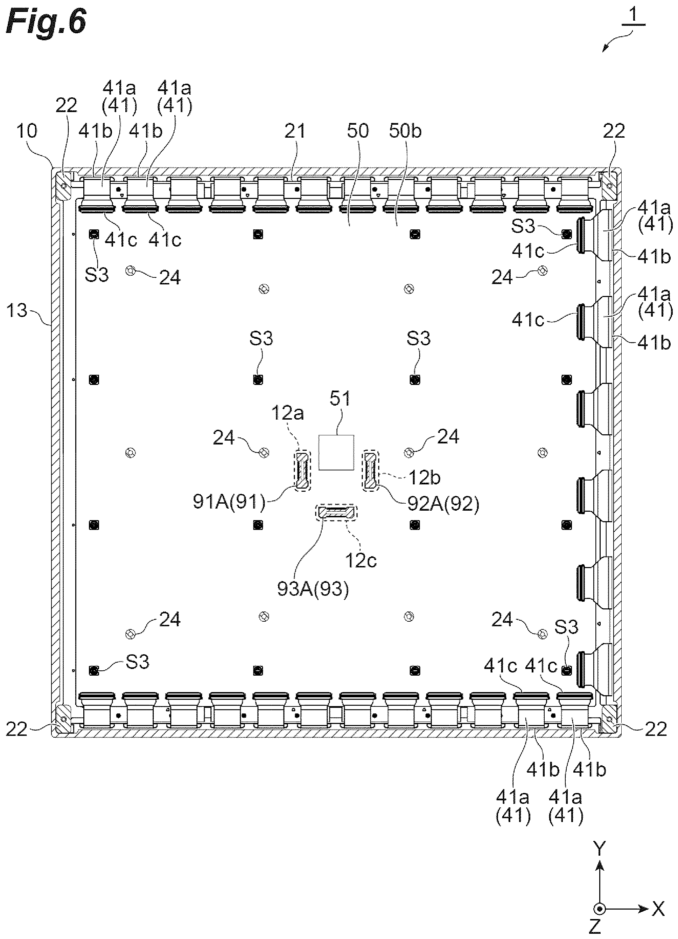

The radiation imaging device 1 includes a first FPGA 51 mounted on the back surface 50b of the control board 50. The first FPGA 51 is a device (field programmable gate array (FPGA)) in which gates capable of programming a configuration of a logic circuit by a user are integrated. The first FPGA 51 has a function of controlling the radiation imaging device 1. As illustrated in FIG. 6, the first FPGA 51 is mounted substantially at the center of the back surface 50b when viewed from the Z-axis direction. The first FPGA 51 is electrically connected to each flexible printed circuit 41 by wiring. Since the first FPGA 51 is mounted substantially at the center of the back surface 50b, the wiring lengths between the first FPGA 51 and the flexible printed circuits 41 (that is, the connector provided on the back surface 50b of the control board 50, corresponding to each of the flexible printed circuits 41) can be made close to each other. As a result, the signal transmission timings between the first FPGA 51 and the flexible printed circuits 41 can be brought close to each other. From the viewpoint of bringing the signal transmission timings closer to each other, the position where the first FPGA 51 is mounted on the back surface 50b and the position where the flexible printed circuit 41 is connected to the back surface 50b (that is, the position of the connector) may be adjusted so that the wire lengths between the first FPGA 51 and each flexible printed circuit 41 are the same as each other.

Next, the second housing 70 and the members housed in the second housing 70 will be described with reference to FIGS. 3 to 5.

The second housing 70 is a hollow container having a substantially rectangular parallelepiped outer shape. The second housing 70 includes a bottom wall 71, a side surface portion 72, and a flange 73. The second housing 70 is provided at a position facing the back surface 50b of the control board 50 with the bottom wall 12 of the first housing 10 interposed therebetween. The bottom wall 71, the side surface portion 72, and the flange 73 are formed of, for example, the same metal material (for example, iron, aluminum, stainless steel, and the like) as the bottom wall 12. In the present embodiment, the bottom wall 71, the side surface portion 72, and the flange 73 are formed of aluminum. The bottom wall 71 is a rectangular plate-shaped member. The bottom wall 71 is provided at a position facing the bottom wall 12 of the first housing 10 via a member housed in the second housing 70.

The side surface portion 72 is connected to an outer edge portion of the bottom wall 71 and is formed in a rectangular annular shape. The side surface portion 72 extends along the XZ plane and the YZ plane. That is, the side surface portion 72 extends along the Z-axis direction. Openings 72a, 72b, and 72c arranged along the Y-axis direction are provided on a surface of the side surface portion 72 on one side in the X-axis direction (right side in FIG. 3). The opening 72a is a hole that exposes a part of the interface circuit 84 (for example, a part of the RJ-45 connector) described later to the outside of the second housing 70. The opening 72b is a hole that exposes a connector 88 described later to the outside of the second housing 70. The opening 72c is a hole that exposes a connector 89 described later to the outside of the second housing 70. The openings 72a, 72b, and 72c may be notches.

The flange 73 extends from the edge of the side surface portion 72 toward the outside of the side surface portion 72, and is formed in a rectangular annular shape. The flange 73 extends along the XY plane. The flange 73 is provided with a plurality of (in the present embodiment, 10) through holes 73a.

The shafts of screws S4 (see FIG. 1) are inserted into the through holes 73a and screwed into the screw holes 12f provided in the bottom wall 12, whereby the second housing 70 and the first housing 10 are fixed to each other. The second housing 70 is fixed to the bottom wall 12 of the first housing 10 so as to cover the pillar 14 of the first housing 10 and the communication holes 12a, 12b, and 12c provided in the bottom wall 12. In the X-axis direction, the length of the second housing 70 is shorter than the length of the first housing 10. In addition, in the Y-axis direction, the length of the second housing 70 is also shorter than the length of the first housing 10. That is, when viewed from the Z-axis direction, the dimensions of the second housing 70 are smaller than the dimensions of the first housing 10, and the second housing 70 (side surface portion 72) is located inside the outer edge of the first housing 10 when viewed from the Z-axis direction.

As illustrated in FIGS. 3 to 5, the radiation imaging device 1 includes circuit boards 81 and 82, a power supply circuit 83, an interface circuit 84, a second FPGA 85, a plurality of (in the present embodiment, 4) pillars 86, a plurality of (in the present embodiment, 2) connectors 87 electrically connecting the circuit boards 81 and 82 to each other, and connectors 88 and 89 connected to the outside, which are housed in a second housing 70.

The circuit board 81 is formed in a rectangular plate shape. The power supply circuit 83 is mounted on the circuit board 81. The circuit board 81 has a first surface 81a and a second surface 81b opposite to the first surface 81a. The circuit board 81 is accommodated in the second housing 70 such that the first surface 81a faces the bottom wall 12 of the first housing 10 (that is, the second surface 81b faces the bottom wall 71 of the second housing 70). A plurality of (in the present embodiment, 11) through holes 81c are provided in a portion along the outer edge portion of the circuit board 81 and the central portion of the circuit board 81. A shaft of a screw S5 (see FIG. 4) is inserted into a through hole 81c and screwed into the screw hole 14a (see FIG. 3) provided at the tip portion of the pillar 14, whereby the circuit board 81 and the first housing 10 are fixed to each other. As a result, the pillar 14 is in contact with a ground electrode G provided on the first surface 81a of the circuit board 81. For example, the ground electrode G is formed around each through hole 81c, and when the circuit board 81 is fixed to the pillar 14, the tip of the pillar 14 comes into contact with the ground electrode G. That is, the circuit board 81 is fixed to the first housing 10 such that the ground electrode G provided on the circuit board 81 is electrically connected to the first housing 10.

The circuit board 82 is formed in a rectangular plate shape. The interface circuit 84 is mounted on the circuit board 82. The circuit board 82 has a first surface 82a and a second surface 82b opposite to the first surface 82a. The circuit board 82 is accommodated in the second housing 70 such that the first surface 82a faces the bottom wall 71 of the second housing 70 with the circuit board 81 interposed therebetween. In the X-axis direction, the length of the circuit board 82 is shorter than the length of the circuit board 81. In addition, in the Y-axis direction, the length of the circuit board 82 is shorter than the length of the circuit board 81. That is, the dimension of the circuit board 82 is smaller than the dimension of the circuit board 81 when viewed from the Z-axis direction. The first surface 82a faces the first surface 81a of the circuit board 81, and the second surface 82b faces the bottom wall 12 of the first housing 10. The circuit board 82 is fixed to the circuit board 81 via a plurality of pillars 86 (four pillars provided at four corners of the first surface 82a in the present embodiment) provided on the first surface 82a. The pillars 86 are made of a metal material (for example, iron, aluminum, stainless steel, brass, and the like). In the present embodiment, the pillars 86 are formed of brass.

Next, a method of fixing the circuit board 81 and the circuit board 82 to each other will be specifically described. The circuit board 82 is provided with a through hole (not illustrated), and a shaft of a screw (not illustrated) is inserted into the through hole from the second surface 82b side. Then, the screw is screwed into the pillar 86, whereby the pillar 86 is fixed to the circuit board 82. Further, the circuit board 81 is provided with a plurality of (in the present embodiment, 4) through holes 81e, and a shaft of a screw (not illustrated) is inserted into the through hole 81e from the second surface 81b side. Then, the screw is screwed into the pillar 86, whereby the pillar 86 is fixed to the circuit board 81. As a result, the circuit board 81 and the circuit board 82 are fixed to each other. At this time, the tip of at least one pillar 86 comes into contact with an electrode (not illustrated) formed around the corresponding at least one through hole 81e. The electrode is electrically connected, in the circuit board 81, to the ground electrode G electrically connected to the first housing 10. As a result, the circuit board 82 is electrically connected to the first housing 10 via the circuit board 81.

The power supply circuit 83 is a circuit for supplying power to the control board 50. The power supply circuit 83 includes elements such as a DC/DC converter and an inductor coil. The DC/DC converter is a device that converts a DC voltage into a DC voltage by switching control. The inductor coil is a passive element capable of storing energy in a magnetic field formed by a flowing current. The power supply circuit 83 configured as described above can be a noise generation source. The power supply circuit 83 is provided on most of the portion excluding the outer edge portion of the second surface 81b of the circuit board 81.

The interface circuit 84 is a circuit for outputting a signal from the control board 50 (for example, an electric signal corresponding to radiation detected by the sensor panel 30) to an external PC device (for example, a device or the like that displays a radiation image). The interface circuit 84 includes, for example, a device for realizing a physical layer in a network protocol (Ethernet (registered trademark) PHY), a registered jack (RJ)-45 connector which is a connector for Ethernet (registered trademark), and the like. The interface circuit 84 configured as described above can be a noise generation source. The interface circuit 84 is provided at one corner (a lower right corner in FIG. 3) of the first surface 82a of the circuit board 82. A part of the interface circuit 84 (for example, a part of the RJ-45 connector) is located in a notch 81d provided in the circuit board 81 when the circuit board 82 is fixed to the circuit board 81. That is, when the circuit boards 81 and 82 are fixed to each other, the interface circuit 84 is disposed at the position where the notch 81d is provided, so that the interface circuit 84 does not interfere with the circuit board 81. A part of the interface circuit 84 (for example, a part of the RJ-45 connector) is exposed to the outside of the second housing 70 through the opening 72a provided in the side surface portion 72 of the second housing 70.

As illustrated in FIGS. 4 and 5, the circuit boards 81 and 82, the power supply circuit 83, and the interface circuit 84 overlap the central portion of the back surface 50b of the control board 50 when viewed from the Z-axis direction, and do not overlap the outer edge portion of the back surface 50b of the control board 50. With such an arrangement, the circuit boards 81 and 82, the power supply circuit 83, and the interface circuit 84 do not overlap the flexible printed circuit 41 connected to the outer edge portion of the back surface 50b of the control board 50 when viewed from the Z-axis direction.

The second FPGA 85 is a device (field programmable gate array (FPGA)) in which gates capable of programming a configuration of a logic circuit by a user are integrated. The second FPGA 85 has a function of controlling an interface conforming to Gigabit Ethernet (GigE)(registered trademark). As illustrated in FIG. 4, the second FPGA 85 is mounted on the second surface 82b of the circuit board 82. The second FPGA 85 is provided so as not to overlap any of the communication holes 12a to 12c provided in the bottom wall 12 of the first housing 10 when viewed from the Z-axis direction. As a result, even if the second FPGA 85 serves as a noise source, the noise of the second FPGA 85 is prevented from propagating to the control board 50 side via the communication holes 12a to 12c. In the present embodiment, the second FPGA 85 is arranged at a position not overlapping with the first FPGA 51 when viewed from the Z-axis direction.

The connector 87 is connected to the first surface 81a of the circuit board 81 and the first surface 82a of the circuit board 82. Signals are transmitted and received between the circuit boards 81 and 82 via the connector 87.

The connectors 88 and 89 are provided at an end portion of the first surface 81a of the circuit board 81 on one side (left side in FIG. 5) in the X-axis direction. The connector 88 is exposed to the outside of the second housing 70 through the opening 72b provided in the side surface portion 72 of the second housing 70. The connector 89 is exposed to the outside of the second housing 70 through the opening 72c provided in the side surface portion 72 of the second housing 70. The connectors 88 and 89 are located inside the outer edge of the first housing 10 when viewed from the Z-axis direction.

The radiation imaging device 1 includes connection members 91, 92, and 93 for electrically connecting the control board 50 and the circuit boards 81 and 82. The connection members 91 to 93 will be described in detail with reference to FIGS. 4 to 6. The connection member 91 (first connection member) includes a pair of connection members 91A and 91B. The connection members 91A and 91B are BtoB connectors (board-to-board connectors configured by a plurality of pins) that can be connected to each other along the Z-axis direction. The connection member 92 (second connection member) includes a pair of connection members 92A and 92B. The connection members 92A and 92B are BtoB connectors that can be connected to each other along the Z-axis direction. The connection member 93 includes a pair of connection members 93A and 93B. The connection members 93A and 93B are BtoB connectors that can be connected to each other along the Z-axis direction.

The connection members 91A, 92A, and 93A are connected to a substantially central portion of the back surface 50b of the control board 50. The connection member 91A is disposed on the first side with respect to the first FPGA 51 (in the present embodiment, one side in the X-axis direction (left side in FIG. 6)) when viewed from the Z-axis direction. The connection member 92A is disposed on the second side different from the first side with respect to the first FPGA 51 (in the present embodiment, the other side in the X-axis direction (right side in FIG. 6)) when viewed from the Z-axis direction. In the present embodiment, as an example, the connection members 91A and 92A sandwich the first FPGA 51 in the X-axis direction. Specifically, the connection members 91A and 92A sandwich a lower half of the first FPGA 51 (lower side in FIG. 6) in the X-axis direction. In the present embodiment, as described above, the distances between the first FPGA 51 and the connection members 91A and 92A in plan view are assumed to be substantially the same, so that the wiring length between the first FPGA 51 and the connection member 91A and the wiring length between the first FPGA 51 and the connection member 92A are assumed to be substantially the same. The connection member 93A is located between the connection members 91A and 92A in the X-axis direction. The connection member 93A is spaced apart from the connection members 91A and 92A on one side in the Y axis direction (lower side in FIG. 6). The connection members 91A to 93A are arranged in a C shape surrounding the first FPGA 51 when viewed from the Z-axis direction. That is, the connection members 91A to 93A are arranged to be point-asymmetric with the first FPGA 51 as a center when viewed from the Z-axis direction.

The connection members 91B, 92B, and 93B are connected to the first surface 81a of the circuit board 81. The connection members 91B, 92B, and 93B are disposed at positions corresponding to the connection members 91A, 92A, and 93A, respectively. In the present embodiment, the connection members 91B to 93B are arranged in a C shape so as to overlap the connection members 91A to 93A, respectively, when viewed from the Z-axis direction.

Next, roles of the connection members 91 to 93 will be described. Image data and a universal asynchronous receiver-transmitter (UART) signal are transmitted and received between the control board 50 and the second FPGA 85 via the connection members 91 and 92. Signals for controlling the LED for displaying the status state is transmitted from the control board 50 to the circuit board 81 via the connection member 93. I/O signals are transmitted and received between the first FPGA 51 and the external device via the connection member 93 and the connector 89. A signal for controlling the power supply circuit 83 is transmitted from the control board 50 to the power supply circuit 83 via the connection member 93.

Next, a positional relationship between the communication holes 12a to 12c and each member in the radiation imaging device 1 will be described. In FIG. 6, the communication holes 12a to 12c are schematically illustrated. As illustrated in FIG. 6, the communication holes 12a to 12c are provided, when viewed from the Z-axis direction, so as not to overlap with a portion 41a of the flexible printed circuit 41 from an end portion 41b farthest from the control board 50 (that is, the folded end portion of the flexible printed circuit 41) in the X-axis direction or the Y-axis direction (second direction) orthogonal to the Z-axis direction to an end portion 41c connected to the control board 50 (connector provided on the back surface 50b). As illustrated in FIGS. 4 and 5, the flexible printed circuit 41 extends from the first surface 31a of the sensor board 31 toward the side wall 13 of the first housing 10, is curved on the way, and extends toward the back surface 50b of the control board 50. By bending the flexible printed circuit 41 in this manner, the folded end portion 41b of the flexible printed circuit 41 is formed on the side of the control board 50 as illustrated in FIG. 6. In other words, the end portion 41b of one flexible printed circuit 41 is an end of the flexible printed circuit 41 that is farthest from a side surface of the control board 50 facing the flexible printed circuit 41 in the direction perpendicular to the side surface. The portion 41a of the flexible printed circuit 41 is a portion extending from the end portion 41b to a connection portion (connector) of the back surface 50b of the control board 50. That is, the portion 41a is a portion that can be visually recognized when the flexible printed circuit 41 is viewed along the Z-axis direction from the bottom wall 12 side. In the present embodiment, the communication holes 12a to 12c are provided so as not to overlap with the entirety of each flexible printed circuits 41 when viewed from the Z-axis direction.

As illustrated in FIGS. 4 and 5, the communication holes 12a to 12c are located outside a region R linearly connecting the flexible printed circuit 41 and the power supply circuit 83. The region R linearly connecting the flexible printed circuit 41 and the power supply circuit 83 is a set of straight lines connecting an arbitrary point on the flexible printed circuit 41 and an arbitrary point on the power supply circuit 83. FIGS. 4 and 5 schematically illustrate the outer edge of the cross section of the region R. As described above, since the communication holes 12a to 12c are located outside the region R, even when noise generated from the power supply circuit 83 radially propagates, the noise directed to the flexible printed circuit 41 is shielded by the bottom wall 12 of the first housing 10, and thus, it is possible to further suppress the influence of the noise on the flexible printed circuit 41. Further, in the present embodiment, the communication holes 12a to 12c are also located outside a region (not illustrated) linearly connecting the flexible printed circuit 41 and the interface circuit 84. The region is a set of straight lines connecting an arbitrary point on the flexible printed circuit 41 and an arbitrary point on the interface circuit 84. As a result, even when noise generated from the interface circuit 84 radially propagates, the noise directed to the flexible printed circuit 41 is shielded by the bottom wall 12 of the first housing 10, so that the influence of the noise on the flexible printed circuit 41 can be suppressed. In addition, from the viewpoint of more effectively suppressing the influence of noise caused by the circuit elements (the power supply circuit 83, the interface circuit 84, and the like) included in the circuit board 81 described above, the communication holes 12a to 12c may be located outside a region linearly connecting the flexible printed circuit 41 and the circuit board 81. Similarly, the communication holes 12a to 12c may be located outside a region linearly connecting the flexible printed circuit 41 and the circuit board 82.

### [Operations and Effects]

In the radiation imaging device 1 described above, the main surface 50a of the control board 50 faces the sensor panel 30, and the second housing 70 accommodating the circuit boards 81 and 82 faces the back surface 50b of the control board 50 with the bottom wall 12 of the first housing 10 interposed therebetween. That is, the control board 50, the circuit board 81 on which the power supply circuit 83 is mounted, and the circuit board 82 on which the interface circuit 84 is mounted are arranged side by side along the Z-axis direction with respect to the sensor panel 30. As a result, the dimensions in plan view (that is, an area occupied by the radiation imaging device 1 when viewed from the Z-axis direction) can be reduced as compared with a case where the control board 50 and the circuit boards 81 and 82 are arranged side by side along the direction perpendicular to the Z-axis direction with respect to the sensor panel 30 (for example, X-axis direction or Y-axis direction). Furthermore, the communication holes 12a to 12c provided in the bottom wall 12 are provided so as not to overlap with the portion 41a of the flexible printed circuit 41 when viewed from the Z-axis direction. As a result, it is possible to suppress noise generated from the power supply circuit 83 and the interface circuit 84 from propagating to the flexible printed circuit 41 via the communication holes 12a to 12c. As a result, deterioration in image quality of the radiation image or the like caused by the noise is suppressed, so that the reliability of the radiation imaging device 1 can be improved. Therefore, according to the configuration described above, it is possible to achieve both improvement in reliability of the radiation imaging device 1 and reduction in dimensions of the radiation imaging device 1 in a plan view.

Further, in the present embodiment, the communication holes 12a to 12c are provided so as not to overlap with the entirety of each flexible printed circuits 41 when viewed from the Z-axis direction. That is, the communication holes 12a to 12c are provided so as not to overlap not only the portion 41a directly facing the bottom wall 12 in the Z-axis direction but also the portion 41d located on the opposite side to the portion 41a with respect to the end portion 41b (that is, the portion from the end portion 41b to the connection portion of the first surface 31a of the sensor board 31). Here, not only the bottom wall 12 but also each member supported by the support member 20 is interposed between the portion 41d and the circuit boards 81 and 82. Therefore, if the connection portion between the portion 41d and the sensor board 31 (first surface 31a) is provided so as to be located inside the connection portion between the portion 41a and the control board 50 (back surface 50b), even if the communication holes 12a to 12c are provided so as to overlap the portion 41d while not overlapping the portion 41a in the Z-axis direction, it is considered that the degree to which the radiation noise from the circuit elements mounted on the circuit boards 81 and 82 affects the portion 41d is low. On the other hand, when the communication holes 12a to 12c are provided so as to overlap the portion 41d as described above, it cannot be said that there is no influence of the noise on the portion 41d. Therefore, according to the configuration in which the communication holes 12a to 12c are provided so as not to overlap the entire flexible printed circuit 41 (that is, both the portion 41a and the portion 41d) as described above, it is possible to more effectively suppress the noise generated from the power supply circuit 83 and the interface circuit 84 from propagating to the flexible printed circuit 41 (the portion 41a and the portion 41d) via the communication holes 12a to 12c. Therefore, the reliability of the radiation imaging device 1 can be further improved.

In addition, the radiation imaging device 1 includes the connection members 91 to 93, and the communication holes 12a to 12c are provided to correspond to the connection members 91 to 93, respectively. According to the configuration described above, it is possible to suppress noise generated from the power supply circuit 83 and the interface circuit 84 from propagating to the flexible printed circuit 41 via the communication holes 12a to 12c while increasing the amount of signal transmission between the control board 50 and the circuit boards 81 and 82.

In addition, the first FPGA 51 is mounted on the control board 50, and the communication holes 12a to 12c are provided so as not to overlap with the first FPGA 51 when viewed from the Z-axis direction. According to the configuration described above, it is possible to suppress noise generated from the power supply circuit 83 and the interface circuit 84 from propagating to the first FPGA 51 via the communication holes 12a to 12c. Thereby, reliability of the first FPGA 51 can be improved, and malfunction of the first FPGA 51 due to the noise can be suppressed.

In addition, the connection member 91 (connection member 91A) is arranged on the first side with respect to the first FPGA 51 when viewed from the Z-axis direction, and the connection member 92 (connection member 92A) is arranged on the second side different from the first side with respect to the first FPGA 51. In the present embodiment, as an example, the connection member 91 and the connection member 92 are arranged to face each other with the first FPGA 51 interposed therebetween. According to the above configuration, since the wiring length between the first FPGA 51 and the connection member 91 (connection member 91A) and the wiring length between the first FPGA 51 and the connection member 92 (connection member 92A) can be made substantially the same, the timing of signal transmission/reception between the first FPGA 51 and the connection member 91 (connection member 91A) and the timing of signal transmission/reception between the first FPGA 51 and the connection member 92 (connection member 92A) can be made substantially the same.

In addition, the connection members 91 to 93 (connection members 91A to 93A) are arranged to be point-asymmetric with the first FPGA 51 as a center when viewed from the Z-axis direction. According to the configuration described above, when the control board 50 and the circuit board 81 are connected to each other by the connection members 91 to 93, the relative orientation of the control board 50 and the circuit board 81 can be easily specified based on the arrangement of the connection members 91 to 93. Therefore, the efficiency of connecting the control board 50 and the circuit board 81 via the connection members 91 to 93 can be improved.

In addition, the communication holes 12a to 12c are located outside the region R linearly connecting the flexible printed circuit 41 and the power supply circuit 83. According to the configuration described above, it is possible to more effectively suppress noise generated from the power supply circuit 83 from propagating to the flexible printed circuit 41 via the communication holes 12a to 12c. As a result, the reliability of the radiation imaging device 1 can be further improved.

In addition, the radiation imaging device 1 includes connectors 88 and 89 provided on the circuit board 81. The second housing 70 has a side surface portion 72 extending in the Z-axis direction and provided with openings 72b and 72c for exposing the connectors 88 and 89 to the outside of the second housing 70, respectively. The side surface portion 72 is located inside the outer edge of the first housing 10 when viewed from the Z-axis direction. According to the configuration described above, the radiation imaging device 1 (circuit board 81) can be connected to the external device via the respective connectors 88 and 89 exposed to the outside via the respective openings 72b and 72c of the side surface portion 72 of the second housing 70. Furthermore, since the side surface portion 72 is located inside the outer edge of the first housing 10 when viewed from the Z-axis direction, it is possible to suppress an increase in dimensions of the radiation imaging device 1 in plan view while adopting the configuration in which the connectors 88 and 89 are exposed from the side surface portion 72 as described above. In addition, by providing the connection ports (openings) of the connectors 88 and 89 in the side surface portion 72 instead of the bottom wall 71 of the second housing 70, it is also possible to suppress an increase in length of the entire device along the Z-axis direction.

Further advantages of the configuration in which the connectors 88 and 89 are exposed from the side surface portion 72 of the second housing 70 as described above will be specifically described. The radiation imaging device 1 is mounted on, for example, dental computed tomography (CT). In the case of imaging up to the tip of the jaw of a patient using the CT, if the dimensions of the radiation imaging device 1 in plan view increase, the radiation imaging device 1 may come into contact with the shoulder of the patient. Conventionally, for example, since the connectors 88 and 89 are exposed from the side wall 13 of the first housing 10, it is necessary to devise and route the cable so as not to increase the dimensions of the entire device including the cable connected to the connectors 88 and 89 in plan view. On the other hand, in the radiation imaging device 1, it is easy to arrange the cable in a space outside the side surface portion 72 and inside the outer edge (side wall 13) of the first housing 10 so that the cable does not protrude outside the outer edge of the first housing 10 (alternatively, in order to reduce the amount of protrusion even when protruding). That is, according to the radiation imaging device 1, while adopting the configuration in which the connectors 88 and 89 are exposed from the side surface portion 72, an increase in the dimensions of the radiation imaging device 1 in plan view can be suppressed, and the routing of the cable can be facilitated by using the space.

In the radiation imaging device 1, the openings 72b and 72c are provided in the side surface portion 72, but the positions where the openings 72b and 72c are provided are not limited to the side surface portion 72. That is, it is sufficient if the openings 72b and 72c may be provided in the second housing 70. For example, the openings 72b and 72c may be provided in the bottom wall 71. Even in this case, it is possible to suppress an increase in the dimensions of the radiation imaging device 1 in plan view while adopting the configuration in which the connectors 88 and 89 are exposed from the second housing 70.

In addition, the circuit board 81 is fixed to the first housing 10 (pillar 14) such that the ground electrode G provided on the circuit board 81 is electrically connected to the first housing 10 (pillar 14). According to the configuration described above, when the first housing 10 is set to the ground potential, the circuit board 81 can be easily connected to GND. Further, by fixing the circuit board 81 to the first housing 10 via the pillar 14, the positional relationship between the control board 50 housed in the first housing 10 and the circuit board 81 housed in the second housing 70 can be stabilized.

Further, the circuit boards 81 and 82 are disposed so as not to overlap with the flexible printed circuit 41 when viewed from the Z-axis direction. As described above, by arranging the circuit boards 81 and 82 themselves including the circuit element serving as the noise source so as not to overlap the flexible printed circuit 41 in the Z-axis direction, it is possible to more effectively suppress the noise caused by the circuit boards 81 and 82 from propagating to the flexible printed circuit 41. As a result, the reliability of the radiation imaging device 1 can be further improved.

[Second Embodiment]

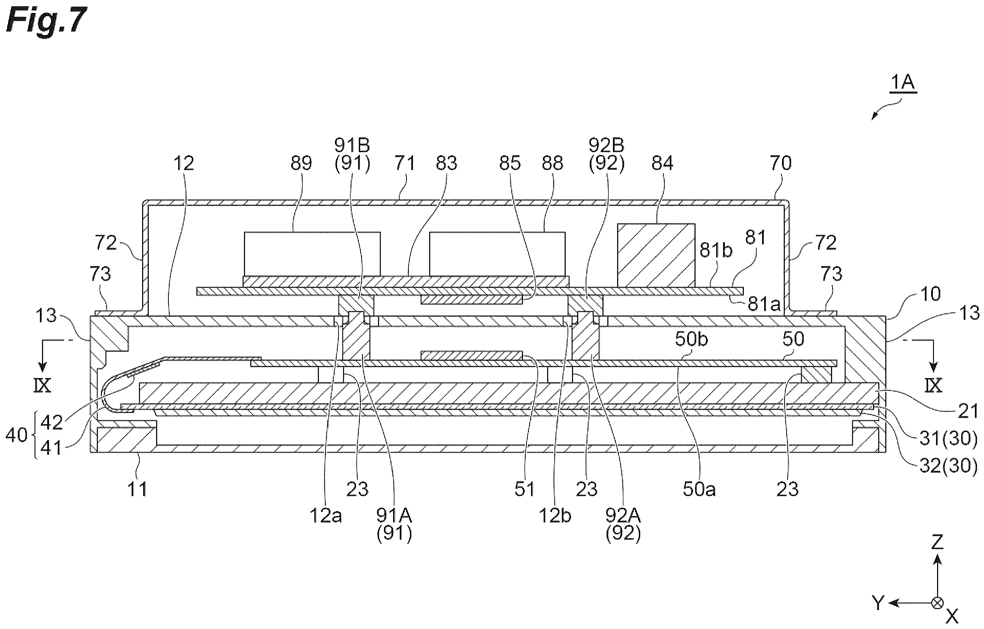

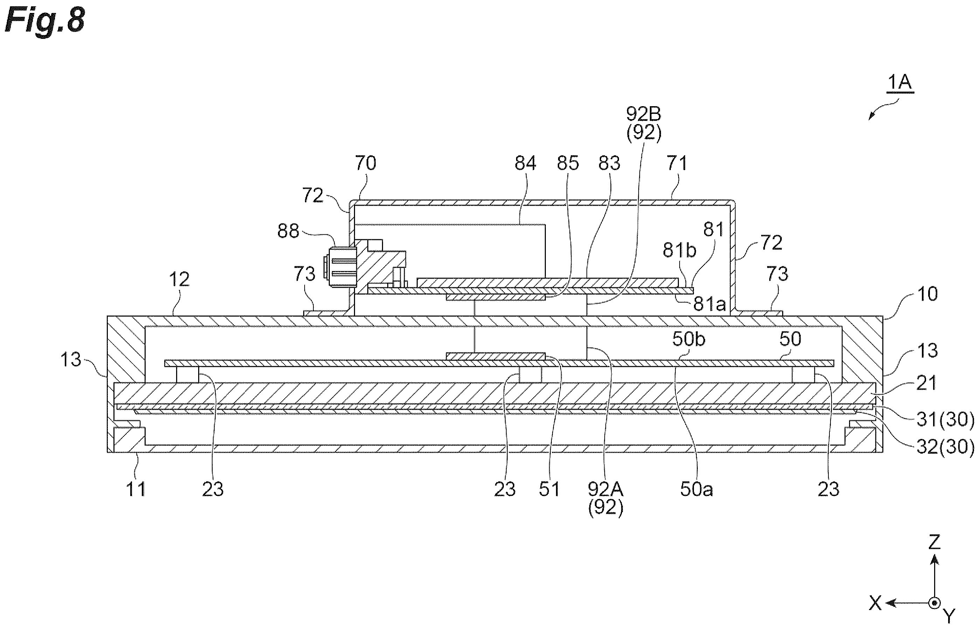

A configuration of a radiation imaging device 1A according to a second embodiment will be described with reference to FIGS. 7, 8, and 9. The radiation imaging device 1A is different from the radiation imaging device 1 in that the radiation imaging device 1A does not include the circuit board 82 (that is, the number of circuit boards is one), that the second FPGA 85 overlaps the first FPGA 51 when viewed from the Z-axis direction, and that the radiation imaging device 1A does not include the connection member 93 (that is, the number of connection members is two). Hereinafter, a part of the configuration of the radiation imaging device 1A different from the radiation imaging device 1 will be mainly described.

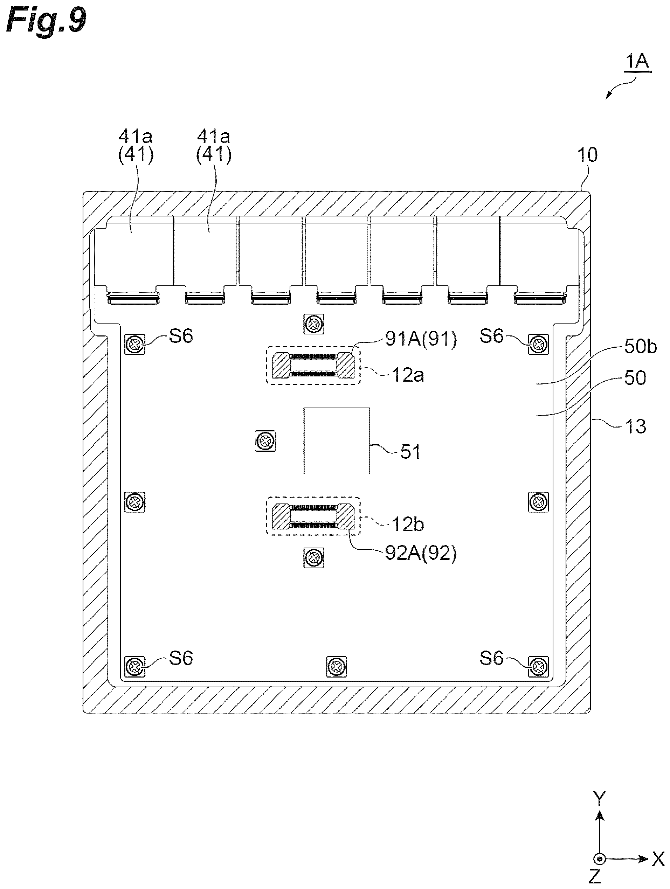

The radiation imaging device 1A includes a plurality of (in the present embodiment, 7) flexible printed circuits 41 (flexible circuit boards 40). In the present embodiment, the seven flexible printed circuits 41 are connected to an outer edge portion of back surface 50b of the control board 50 on one side (upper side in FIG. 9) in the Y-axis direction at substantially equal intervals.

The power supply circuit 83 and the interface circuit 84 are provided on the second surface 81b of the circuit board 81. The second FPGA 85 is provided on the first surface 81a of the circuit board 81. That is, in the present embodiment, the power supply circuit 83, the interface circuit 84, and the second FPGA 85 are provided on one circuit board 81. In the present embodiment, at least a part (in the present embodiment, substantially all of) of the second FPGA 85 is arranged so as to overlap the first FPGA 51 when viewed from the Z-axis direction.

The connection members 91B and 92B are connected to the first surface 81a of the circuit board 81. The connection members 91B and 92B are disposed at positions corresponding to the connection members 91A and 92A, respectively. The connection members 91B and 92B sandwich the second FPGA 85 in the Y-axis direction. In the present embodiment, the connection members 91B and 92B are arranged so as to overlap the connection members 91A and 92A, respectively, when viewed from the Z-axis direction.

In the radiation imaging device 1A, image data and UART signals are transmitted and received between the control board 50 and the second FPGA 85 via the connection member 91. A signal for controlling the LED for displaying the status state is transmitted from the control board 50 to the circuit board 81 via the connection member 92. I/O signals are transmitted and received between the first FPGA 51 and the external device via the connection member 92 and the connector 89. A signal for controlling the power supply circuit 83 is transmitted from the control board 50 to the power supply circuit 83 via the connection member 92.

Next, a positional relationship between the communication holes 12a and 12b and each member in the radiation imaging device 1A will be described. In FIG. 9, the communication holes 12a and 12b are schematically illustrated. Similarly to the first embodiment, the communication holes 12a and 12b are provided so as not to overlap with the portion 41a of each flexible printed circuit 41 when viewed from the Z-axis direction. In the present embodiment, similarly to the first embodiment, the communication holes 12a and 12b are provided so as not to overlap with the entirety of each flexible printed circuit 41 when viewed from the Z-axis direction.

Also in the radiation imaging device 1A described above, regarding the configuration common to the radiation imaging device 1 according to the first embodiment, the same effects as those of the radiation imaging device 1 according to the first embodiment are obtained.

In addition, in the radiation imaging device 1A, at least a part of the second FPGA 85 overlaps the first FPGA 51 when viewed from the Z-axis direction. According to the configuration described above, the connection members 91 and 92 connecting the control board 50 and the circuit board 81 to each other along the Z-axis direction can be connected to the back surface 50b of the control board 50 in the vicinity of the first FPGA 51 and can be connected to the circuit board 81 in the vicinity of the second FPGA 85. Accordingly, since the wiring length between the first FPGA 51 and the second FPGA 85 can be shortened, it is possible to suppress the occurrence of delay or rounding of the transmission signal waveform between the first FPGA 51 and the second FPGA 85.

### [Modifications]

The present disclosure is not limited to some embodiments described above. The material and shape of each configuration described above are not limited to the material and shape described above, and various materials and shapes can be adopted. In addition, some configurations included in the above embodiments may be omitted or changed as appropriate. For example, some characteristic configurations and some effects exhibited by each configuration included in each of the above embodiments have been described, but the radiation imaging device according to the present disclosure does not necessarily need to be configured to exhibit all the effects described in each of the above embodiments, and may be configured to exhibit only some of the effects described in each of the above embodiments. In the latter case, the radiation imaging device is only required to have a configuration essential for exerting at least the partial effect, and a configuration that is not essential for exerting the partial effect may be omitted or changed as appropriate. Note that, in a case where one effect is focused on, the configuration essential for exerting the one effect should be reasonably grasped based on the technical common sense and the description of the present specification on the basis of those skilled in the art. Hereinafter, some specific modifications of the radiation imaging device according to the present disclosure will be illustrated.

In each of the embodiments described above, the flexible printed circuit 41 extends from the first surface 31a of the sensor board 31 toward the side wall 13 of the first housing 10, is curved on the way, and extends toward the back surface 50b of the control board 50, however, the flexible printed circuit 41 is not limited to the above embodiment. For example, the flexible printed circuit 41 may extend substantially linearly.

In the second embodiment, the connection members 91A and 92A sandwich the first FPGA 51 in the Y-axis direction, but the arrangement of the connection members 91A and 92A is not limited to the above embodiment. For example, the connection member 91A may be disposed on one side (the left side in FIG. 9) in the X-axis direction with respect to the first FPGA 51, and the connection member 92A may be disposed on one side (the lower side in FIG. 9) in the Y-axis direction with respect to the first FPGA 51. That is, the connection members 91A and 92A may be arranged in an L shape when viewed from the Z-axis direction. Even in this case, the wiring length between the first FPGA 51 and the connection member 91 (connection member 91A) and the wiring length between the first FPGA 51 and the connection member 92 (connection member 92A) can be made substantially the same.

In the first embodiment, the control board 50 and the circuit board 81 are connected by the connection members 91 to 93, and in the second embodiment, the control board 50 and the circuit board 81 are connected by the connection members 91 and 92, but the number of connection members connecting the control board 50 and the circuit board 81 may be one or four or more. The number of communication holes provided in the bottom wall 12 may be determined according to the number of connection members, and may be one or four or more. In addition, each of the connection members 91 to 93 is a separation-type connector formed in a pillar shape extending along the Z-axis direction, but each of the connection members 91 to 93 may be an integrated connection member. In this case, each of the connection members 91 to 93 may be formed of, for example, a flexible printed circuit. In addition, in each of the embodiments described above, the connection member and the communication hole have a one-to-one relationship, but a plurality of connection members may pass through one communication hole.

In each of the embodiments described above, the flange 73 is provided at the edge of the side surface portion 72 of the second housing 70, and the opening is formed by the edge of the side surface portion 72, but the second housing 70 may be a box-shaped member in which the opening is closed. That is, the second housing 70 may have a wall portion provided at a position facing the bottom wall 71 to close the opening. In such a case, it is sufficient that the wall portion is provided with, for example, communication holes communicating with the communication holes 12a to 12c provided in the bottom wall 12 of the first housing 10.

A sheet for shielding electromagnetic waves (noise) may be provided in the first housing 10 or the second housing 70. For example, a sheet for shielding electromagnetic wave may be provided on both or any one of the inner surface and the outer surface of the bottom wall 12 of the first housing 10. In addition, in a case where the second housing 70 includes the above-described wall portion (wall portion facing the bottom wall 12), a sheet for shielding electromagnetic wave may be provided on both or any one of the inner surface and the outer surface of the wall portion of the second housing 70. By providing the sheet having the electromagnetic wave shielding effect on the wall portion of the first housing 10 or the second housing 70 disposed between the control board 50 and the circuit board 81 as described above, the influence of noise on the flexible printed circuit 41 can be more effectively suppressed.

### Reference Signs List

- 1, 1A: Radiation imaging device
- 10: First housing
- 12: Bottom wall (wall portion)
- 12a to 12c: Communication hole (at least one communication hole)
- 30: Sensor panel
- 41: Flexible printed circuit
- 41a: Portion
- 41b, 41c: End portion
- 50: Control board
- 50a: Main surface
- 50b: Back surface
- 51: First FPGA
- 70: Second housing
- 72: Side surface portion
- 72b, 72c: Opening
- 81, 82: Circuit board
- 83: Power supply circuit
- 84: Interface circuit
- 85: Second FPGA
- 88, 89: Connector
- 91: Connection member (first connection member)
- 92: Connection member (second connection member)
- 93: Connection member
- 91A, 91B, 92A, 92B, 93A, 93B: Connection member
- G: Ground electrode
- R: Region

## Claims

1. A radiation imaging device comprising:
a sensor panel configured to detect radiation;
a control board having a main surface facing the sensor panel along a first direction orthogonal to the sensor panel and a back surface opposite to the main surface, and configured to control reading of a signal from the sensor panel;
a flexible printed circuit disposed so as to connect the sensor panel and the back surface of the control board through a side of the control board when viewed from the first direction;
a circuit board with a power supply circuit that supplies power to the control board and an interface circuit that outputs a signal from the control board to the outside mounted thereon;
a first housing accommodating the sensor panel, the control board, and the flexible printed circuit, and having a wall portion facing the back surface of the control board;
a second housing provided at a position facing the back surface of the control board with the wall portion interposed therebetween and accommodating the circuit board; and
at least one connection member electrically connecting the control board and the circuit board, wherein
the wall portion is provided with at least one communication hole that allows the inside of the first housing and the inside of the second housing to communicate with each other,
the at least one connection member is connected to the control board and the circuit board via the at least one communication hole, and
when viewed from the first direction, the at least one communication hole is provided so as not to overlap a portion of the flexible printed circuit from an end farthest from the control board in a second direction orthogonal to the first direction to an end connected to the control board.

2. The radiation imaging device according to claim **1,** wherein
the at least one communication hole is provided so as not to overlap the entire flexible printed circuit when viewed from the first direction.

3. The radiation imaging device according to claim 1 or 2, wherein
the at least one connection member includes a plurality of connection members, and
the at least one communication hole includes a plurality of communication holes provided to correspond to the plurality of connection members, respectively.

4. The radiation imaging device according to any one of claims 1 to 3, wherein
a first FPGA is mounted on the control board, and
the at least one communication hole is arranged not to overlap the first FPGA when viewed from the first direction.

5. The radiation imaging device according to claim 4, wherein
the at least one connection member includes a plurality of connection members, and
the plurality of connection members include:
a first connection member disposed on a first side with respect to the first FPGA when viewed from the first direction; and
a second connection member disposed on a second side different from the first side with respect to the first FPGA.

6. The radiation imaging device according to claim 5, wherein
the plurality of connection members are arranged to be point-asymmetric with the first FPGA as a center when viewed from the first direction.

7. The radiation imaging device according to any one of claims 4 to 6, wherein
a second FPGA is mounted on the circuit board, and
at least a part of the second FPGA is arranged to overlap the first FPGA when viewed from the first direction.

8. The radiation imaging device according to any one of claims 1 to 7, wherein
the at least one communication hole is arranged outside a region linearly connecting the flexible printed circuit and the power supply circuit.

9. The radiation imaging device according to any one of claims 1 to 8, further comprising:
a connector provided on the circuit board and connected to the outside, wherein
the second housing is provided with an opening for exposing the connector to the outside of the second housing, and
the connector is located inside an outer edge of the first housing when viewed from the first direction.

10. The radiation imaging device according to claim 9, wherein
the second housing has a side surface portion extending in the first direction and provided with the opening, and
the side surface portion is located inside an outer edge of the first housing when viewed from the first direction.

11. The radiation imaging device according to any one of claims 1 to 10, wherein
the circuit board is fixed to the first housing such that a ground electrode provided on the circuit board is electrically connected to the first housing.

12. The radiation imaging device according to any one of claims 1 to 11, wherein
the circuit board is provided so as not to overlap the flexible printed circuit when viewed from the first direction.
